# EUROPÄISCHE PATENTSCHRIFT

(11) **EP 2 436 325 B1**
(45) Veröffentlichungstag und Bekanntmachung des Hinweises auf die Patenterteilung: **27.11.2013**
(21) Anmeldenummer: 10186117.7
(22) Anmeldetag: 01.10.2010
(51) Int. Cl.: A61B 17/70

(54) **Wirbelsäulenimplantat zur Stabilisierung und Versteifung von Wirbelkörpern**
Spinal implant for stabilising and reinforcing spinal bodies
Implant de colonne vertébrale pour la stabilisation et le raidissement de vertèbres

(43) Veröffentlichungstag der Anmeldung: 04.04.2012
(73) Patentinhaber: Spinelab AG, 8406 Winterthur (CH)
(72) Erfinder: Braunschweiler, Reto, 8413, Neftenbach (CH); Fischli, Simon, 6010, Kriens (CH)
(74) Vertreter: Scheuzger, Beat Otto

(56) Entgegenhaltungen:
- EP-A1- 1 815 812
- EP-A1- 1 961 392
- WO-A1-95/19149
- WO-A1-2006/066685
- FR-A1- 2 752 719

## Beschreibung

Die vorliegende Erfindung bezieht sich auf ein Wirbelsäulenimplantat zur Stabilisierung und Versteifung von Wirbelkörpern einer Wirbelsäule, umfassend erste Knochenschrauben, bestehend aus einem Einschraubteil, welcher in einen Wirbelkörper einschraubbar ist, und ersten Aufnahmemitteln, zweite Knochenschrauben, bestehend aus einem Einschraubteil, welcher in einen Wirbelkörper einschraubbar ist, und zweiten Aufnahmemitteln, erste Verbindungselemente, die starr sind und in die ersten Aufnahmemittel der ersten Knochenschrauben einsetzbar und darin befestigbar sind, zweite Verbindungselemente, die elastisch sind und in die zweiten Aufnahmemittel der zweiten Knochenschrauben einsetzbar und darin befestigbar sind, und Kupplungsmittel , mit welchen jeweils ein erstes Verbindungselement und ein zweites Verbindungselement miteinander verbindbar sind, welche Kupplungsmittel mit einem ersten Bereich lösbar an einer zweiten Knochenschraube, in welcher ein Endbereich eines zweiten Verbindungselementes gehalten ist, befestigbar sind und die Kupplungsmittel mit einem dem ersten Bereich gegenüberliegenden zweiten Bereich mit einem Endbereich eines ersten Verbindungselementes verbindbar sind.

Derartige Wirbelsäulenimplantate sind in vielfältiger Weise bekannt. Mit diesen Wirbelsäulenimplantaten wird erreicht, dass bei der Wirbelsäule bereichsweise eine starre Stabilisierung der Wirbelkörper ermöglicht wird, während in anderen Bereichen die Wirbelkörper durch die elastische Ausgestaltung des Systems gestützt und stabilisiert werden, ohne dass eine Versteifung erfolgt. Im Bereich der starren Stabilisierung der Wirbelkörper ist erwünscht, dass ein knöchernes Verwachsen der betroffenen und stabilisierten Wirbelkörper erreicht wird, bei der elastischen Stabilisierung soll keine Versteifung der Wirbelkörper auftreten.

Ein derartiges Wirbelsäulenimplantat zur Stabilisierung und Versteifung von Wirbelkörpern einer Wirbelsäule ist beispielsweise aus der EP-A 1961392 bekannt.

Wenn nach einer gewissen Zeit das Zusammenwachsen und die Verknöcherung der Wirbelkörper miteinander, die durch das Wirbelsäulenimplantat versteift wurden, soweit erfolgt ist, dass die Eigenstabilität gegeben ist, werden die versteifenden Bereiche des Wirbelsäulenimplantats nicht mehr benötigt, sie sind eher überflüssig und können stören. Es wäre deshalb sinnvoll, wenn diese versteifenden Bereiche des Wirbelsäulenimplantats nach dem entsprechenden Verwachsungsvorgang vom elastisch stabilisierenden Bereich vollständig entkoppelt oder gegebenenfalls sogar entfernt werden könnten, während die Bereiche des Wirbelsäulenimplantates, die der Stabilisierung der Wirbelkörper dienen, beibehalten werden sollen, um diese unterstützende und stabilisierende Wirkung weiter ausüben zu können. Dies bedingt aber, dass der versteifende Bereich und der stabilisierende und unterstützende Bereich des Wirbelsäulenimplantates in einfacher Weise von einander entkoppelt werden können, was mit den bekannten Wirbelsäulenimplantaten nicht ermöglicht wird.

Das Dokument W02006/066685 A1 zeigt Wirbelsäulenimplantate, bei welchen starre Stäbe und elastische Stäbe zur Fixierung bzw. Stabilisierung der Wirbelkörper eingesetzt werden, die miteinander verbunden werden können. Eine völlige Entkoppelung der starren Stäbe von den elastischen Stäben ist nicht möglich, eine Herausnahme der starren Stäbe nach einer Verwachsung der fixierten Wirbelkörper ist ebenfalls nicht möglich, ohne dass die benachbarten elastischen Stäbe wieder neu fixiert werden müssen.

Die Aufgabe der vorliegenden Erfindung besteht somit darin, ein Wirbelsäulenimplantat zur Stabilisierung und Versteifung von Wirbelkörpern einer Wirbelsäule so zu gestalten, dass der stabilisierende Bereich, der ein elastisches Verbindungselement aufweist, und der starre Bereich mit einem starren Verbindungselement derart miteinander verbunden sind, dass sie in einfachster Weise voneinander entkoppelbar sind.

Erfindungsgemäss erfolgt die Lösung dieser Aufgabe dadurch, dass der erste Bereich des Kupplungsmittels mit einem Spannelement versehen ist, welches in die zweiten Aufnahmemittel geführt einsetzbar ist und über Spannmittel, die auf die zweiten Aufnahmemittel aufsetzbar sind, gegen das in die zweiten Aufnahmemittel eingesetzte zweite Verbindungselement spannbar ist, und dass der zweite Bereich des Kupplungsmittels als weiteres Spannmittel ausgebildet ist, in welchem ein Endbereich eines ersten Verbindungselementes befestigbar ist.

Durch diese Lösung lässt sich der starre Bereich des Wirbelsäulenimplantates vom stabilisierenden und elastischen Bereich in einfacher Weise entkoppeln, die Verbindung zwischen der zweiten Knochenschraube, die den Endbereich des elastischen zweiten Verbindungselementes bildet, bleibt während des Eingriffs bestehen, am Stabilisierungsvermögen dieses elastischen Bereichs des Wirbelsäulenimplantats ändert sich somit nichts. Das Einsetzen als auch das Entkoppeln dieses Bereichs des Wirbelsäulenimplantats wird erleichtert.

In vorteilhafter Weise sind die zweiten Aufnahmemittel U-förmig ausgebildet und bestehen aus zwei Schenkeln, zwischen welchen die Auflagefläche für das zweite Verbindungselement angeordnet ist und auf welche zwei Schenkel die Spannmittel, bestehend aus einem mit einer ersten Spannschraube versehenes Verriegelungselement, aufsetzbar und mit diesem verriegelbar sind, was den Arbeitsablauf beim operativen Eingriff erleichtert.

In vorteilhafter Weise ist das Spannelement mit Führungsrippen ausgestattet, welche mit an den Schenkeln angebrachten Führungsflächen die Führungen bilden, was einen einfachen Aufbau ergibt.

Einen besonders einfachen Aufbau dieses weiteren Spannmittels wird dadurch erreicht, dass dieses eine U-förmige Aufnahme für das erste Verbindungselement und einen der U-förmigen Aufnahme gegenüberliegenden Gewindeteil für die Aufnahme einer zweiten Spannschraube aufweist.

Eine Verbesserung der Klemmung des ersten Verbindungsmittels mit dem weiteren Spannmittel kann dadurch erreicht werden, dass zwischen die zweite Spannschraube und das erste Verbindungselement ein Klemmelement eingesetzt wird.

Eine weitere vorteilhafte Ausgestaltung der Erfindung besteht darin, dass das Spannelement und das weitere Spannmittel, welche die Kupplungsmittel bilden, lösbar miteinander verbunden sind, was die Handhabung beim jeweiligen operativen Eingriff erleichtert.

In vorteilhafter Weise weist das Spannelement mindestens einen nockenartigen Vorsprung auf, welcher in das weitere Spannmittel hineinragt, wodurch ein sehr einfacher Aufbau dieser Verbindung erreicht wird.

Eine weitere vorteilhafte Ausgestaltung der Erfindung besteht darin, dass der nockenartige Vorsprung mit einer Gewindebohrung versehen ist, in welchen eine weitere Spannschraube einschraubbar ist, welcher nockenartige Vorsprung zwischen die zweite Spannschraube und das erste Verbindungselement des weiteren Spannmittels hineinragt. Dadurch kann die Position des nockenartigen Vorsprungs bezüglich des Verbindungselementes eingestellt werden, wodurch beispielsweise erste Verbindungselemente mit unterschiedlichem Durchmesser eingesetzt werden können.

Eine weitere vorteilhafte Ausgestaltung der Erfindung besteht darin, dass die zweiten Verbindungselemente eine Oberflächenstruktur aufweisen, die aus im Wesentlichen quer zur Längsachse verlaufenden Rippen und Rillen bestehen, und dass die zweiten Aufnahmemittel und die dem zweiten Verbindungselement zugewandte Fläche der Spannmittel mit Rillen und Rippen versehen sind, die den Rippen und Rillen des zweiten Verbindungselementes entsprechen. Dadurch kann in optimaler Weise zwischen dem zweiten Verbindungselement und den zweiten Aufnahmemitteln der zweiten Knochenschraube eine formschlüssige Verbindung erreicht werden.

Ausführungsformen der vorliegenden Erfindung werden nachfolgend anhand der beiliegenden Zeichnungen beispielhaft näher erläutert.

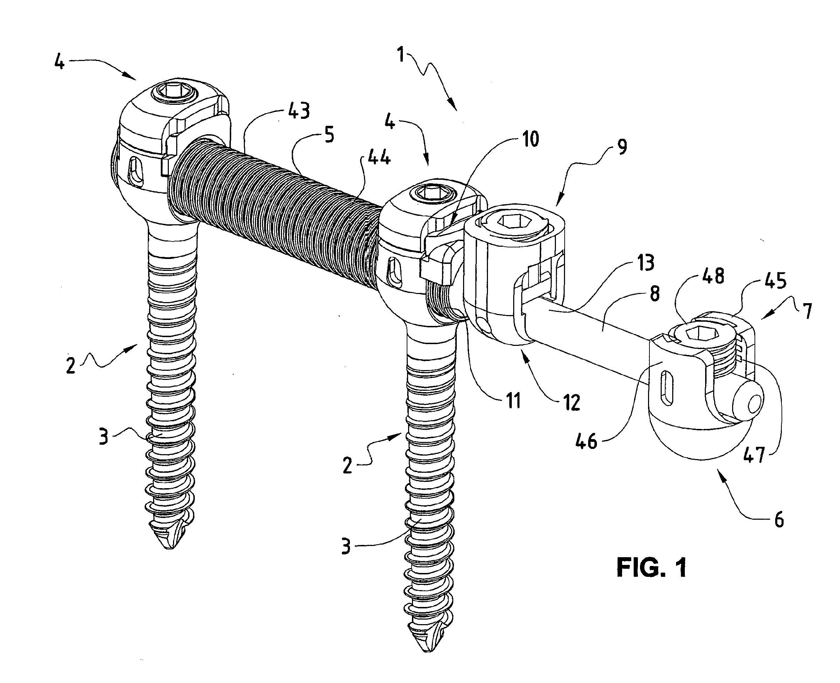

Es zeigt
Fig. 1 in räumlicher Darstellung eine erste Ausführungsform eines Teils eines erfindungsgemässen Wirbelsäulenimplantats;
Fig. 1 in räumlicher Darstellung eine erste Ausführungsform eines Teils eines erfindungsgemässen Wirbelsäulenimplantats;
Fig. 2 in räumlicher Darstellung eine zweite Knochenschraube mit Spannmittel des Wirbelsäulenimplantats gemäss Fig. 1;
Fig. 3 eine Schnittdarstellung durch das Spannmittel gemäss Fig. 2;
Fig. 4 in räumlicher Darstellung das Wirbelsäulenimplantat gemäss Fig. 1 mit in Einzelteilen dargestelltem Kupplungsmittel;
Fig. 5 eine Schnittdarstellung durch das weitere Spannmittel des Wirbelsäulenimplantats gemäss Fig. 1;
Fig. 6 eine Draufsicht auf das räumlich dargestellte Spannelement des Wirbelsäulenimplantats gemäss Fig. 1;
Fig. 7 eine Ansicht von unten auf das räumlich dargestellte Spannelement entsprechend Fig. 6;
Fig. 8 in räumlicher Darstellung eine zweite Ausführungsform eines Teils eines erfindungsgemässen Wirbelsäulenimplantat mit in Einzelteilen dargestelltem Kupplungsmittel;
Fig. 9 eine Schnittdarstellung durch das weitere Spannmittel des Wirbelsäulenimplantats gemäss Fig. 8;
Fig. 10 eine Draufsicht auf das räumlich dargestellte Spannelement des Wirbelsäulenimplantats gemäss Fig. 7;
Fig. 11 eine Ansicht von unten auf das räumlich dargestellte Spannelement entsprechend Fig. 10;
Fig. 12 in räumlicher Darstellung eine dritte Ausführungsform eines Teils eines erfindungsgemässen Wirbelsäulenimplantats mit in Einzelteilen dargestelltem Kupplungsmittel;
Fig. 13 eine Schnittdarstellung durch das weitere Spannmittel des Wirbelsäulenimplantats gemäss Fig. 12;
Fig. 14 eine Draufsicht auf das räumlich dargestellte Spannelement des Wirbelsäulenimplantats gemäss Fig. 12; und
Fig. 15 eine Ansicht von unten auf das räumlich dargestellte Spannelement entsprechend Fig. 14.

Wie aus Fig. 1 ersichtlich ist, setzt sich ein derartiger Teil eines erfindungsgemässen Wirbelsäulenimplantats 1 zur Stabilisierung und Versteifung von Wirbelkörpern einer Wirbelsäule aus zweiten Knochenschrauben 2 zusammen, die jeweils aus einem mit einem Gewinde versehenen Einschraubteil 3 und aus zweiten Aufnahmemitteln 4 bestehen. In bekannter Weise ist eine derartige zweite Knochenschraube 2 mit dem Einschraubteil 3 in einen Wirbelkörper einer Wirbelsäule einschraubbar. In die zweiten Aufnahmemittel 4 ist ein zweites Verbindungselement 5 eingesetzt und festgehalten. Dieses zweite Verbindungselement 5 ist stabförmig ausgebildet und besteht aus einem elastischen Material, beispielsweise aus einem biokompatiblen Kunststoff auf Polyurethan-Basis.

Das erfindungsgemässe Wirbelsäulenimplantat 1 umfasst ferner erste Knochenschrauben 6, welche ebenfalls in bekannter Weise jeweils einen Einschraubteil aufweisen, der in den Figuren nicht dargestellt ist, der aber dem Einschraubteil 3 der zweiten Knochenschraube 2 entspricht und entsprechend in den vorgesehenen Wirbelkörper der Wirbelsäule einschraubbar ist. Diese ersten Knochenschrauben 6 sind mit ersten Aufnahmemitteln 7 ausgestattet, in welche ein erstes Verbindungselement 8, das stangenförmig ausgebildet ist, eingesetzt und befestigt werden kann. Dieses erste Verbindungselement 8 ist starr und besteht aus einem metallischen Werkstoff, beispielsweise einer Titanlegierung.

Das jeweilige erste Verbindungselement 8 ist jeweils mit einem daran angrenzenden zweiten Verbindungselement 5 über Kupplungsmittel 9 verbindbar. Diese Kupplungsmittel 9 sind mit einem ersten Bereich 10 lösbar an einer zweiten Knochenschraube 2 gehalten, in welcher ein Endbereich 11 des zweiten Verbindungsmittels 5 ebenfalls gehalten ist. Der dem ersten Bereich 10 gegenüberliegende zweite Bereich 12 des Kupplungsmittels 9 ist mit einem Endbereich 13 eines ersten Verbindungselements 8 verbindbar, wie später noch im Detail beschrieben wird.

Ein derartiges Wirbelsäulenimplantat 1 ist in praktisch beliebiger Weise an die zu stabilisierende Wirbelsäule anpassbar. Dieses Wirbelsäulenimplantat kann aus starren ersten Verbindungselementen 8 und elastischen zweiten Verbindungselementen 5 zusammengesetzt werden, die mit den jeweiligen ersten bzw. zweiten Knochenschrauben 6 bzw. 2 zusammenwirken. Die jeweiligen Verbindungselemente 8 und 4 können auf das gewünschte Mass abgelängt werden, und durch die entsprechenden Kombinationen kann die Wirbelsäule in den gewünschten Bereichen elastisch stabilisiert und in anderen gewünschten Bereichen versteift werden, wobei in die vorgesehenen Wirbelkörper jeweils die entsprechende Knochenschraube eingeschraubt wird.

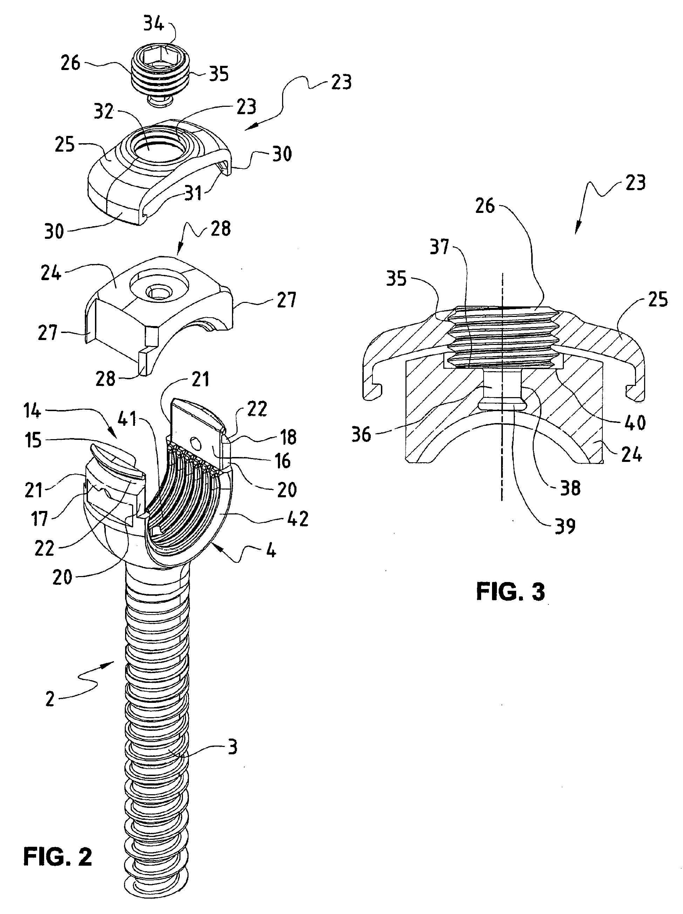

Aus den Fig. 2 und 3 ist der Aufbau der zweiten Knochenschrauben 2 dargestellt. Die mit dem Einschraubteil 3 verbundenen zweiten Aufnahmemittel 4 sind mit einer U-förmigen Ausnehmung 14 versehen, die durch die inneren Oberflächen 15, 16 zweier Schenkel 17, 18 gebildet wird. Die beiden inneren Oberflächen 15 und 16 dieser beiden Schenkel 17 und 18 werden durch eine Grundfläche 19 miteinander verbunden, wobei die inneren Oberflächen 15 und 16 und die Grundfläche 19 der Form des in diese U-förmige Ausnehmung 14 einzusetzenden zweiten Verbindungselements 5 angepasst ist, in welcher Rippen und Rillen 41, 42 vorgesehen sind, in welche entsprechende am zweiten Verbindungselement 5 angebrachte Rillen 43 und Rippen 44 (Fig. 1) im eingesetzten Zustand eingreifen und so eine formschlüssige Verbindung gebildet wird.

Die beiden Seitenbereiche der jeweiligen Schenkel 17 und 18 sind jeweils mit einer Führungsfläche 20, 21 ausgestattet. Im oberen Bereich ist aussenseitig an den beiden Schenkeln 17 und 18 jeweils eine quer zu diesen verlaufende Nut 22 angebracht. Der Nutgrund und die die Nut 22 begrenzenden Flächen sind kreisbogenförmig ausgebildet.

Die U-förmige Ausnehmung 14 der zweiten Aufnahmemittel 4 der zweiten Knochenschraube 2 wird durch Spannmittel 23 verschlossen. Diese Spannmittel 23 setzen sich zusammen aus einem Spannelement 24, einem Verriegelungselement 25 und einer ersten Spannschraube 26. Das Spannelement 24 lässt sich zwischen die beiden Schenkel 17 und 18 einsetzen. Hierzu ist dieses Spannelement 24 mit jeweils zwei einander gegenüberliegenden Führungsrippen 27 und 28 ausgestattet, welche mit den Führungsflächen 20 und 21 der beiden Schenkel 17 und 18 die Führung bilden. Die der Auflagefläche 19 zugewandte Oberfläche 29 des Spannelement 24 weist ebenfalls eine der Form des zweiten Verbindungselements 5 (Fig. 1) angepasste Form auf.

Das Verriegelungselement 25 weist die Form eines Bügels auf und ist an den beiden einander gegenüberliegenden Endbereichen jeweils mit einem Kragen 30 ausgestattet. An jedem dieser Kragen 30 ist ein Nocken 31 angebracht, die gegeneinander gerichtet sind. Zusätzlich ist das Verriegelungselement 25 mit einer Bohrung 32 ausgestattet, welche mit einem Gewinde 33 versehen ist. In das Gewinde 33 dieser Bohrung 32 lässt sich die erste Spannschraube 26 einschrauben, welche mit einem Innensechskant 34 ausgestattet ist. Mit diesen Spannmitteln 23 lässt sich die U-förmige Ausnehmung 14 in den zweiten Aufnahmemitteln 4 der zweiten Knochenschraube 2 verschliessen, das in diese U-förmige Ausnehmung eingelegte zweite Verbindungselement wird dadurch fixiert.

Wie aus Fig. 3 ersichtlich ist, können die Spannmittel 23 zu einer Einheit zusammengesetzt werden. Die erste Spannschraube 26 ist mit einem Gewindeteil 35 versehen, an welchem ein Bolzenstück 36 angesetzt ist. Zwischen dem Gewindeteil 35 und dem Bolzenstück 36 ist eine Abstützfläche 37 gebildet. Das Bolzenstück 36 ragt in eine zentrale Bohrung 38, welche im Spannelement 24 angebracht ist. Der dem Gewindeteil 35 abgewandte Endbereich des Bolzenstücks 36 ist mit einer Verdickung 39 ausgestattet, welche beispielsweise durch Aufkrempen des Endbereichs dieses Bolzenstücks 36 erreichbar ist, welcher Aufkrempvorgang nach dem Einsetzen der ersten Spannschraube 26 in das Spannelement 24 ausgeführt werden kann. Die erste Spannschraube 26 und das Spannelement 24 sind somit um das Bolzenstück 36 verdrehbar miteinander verbunden. Die erste Spannschraube 26 mit dem mit diesem verbundenen Spannelement 24 lässt sich dann in das Verriegelungselement 25 einschrauben, wodurch das Spannmittel 23 als Einheit ausgebildet ist. Zum Aufsetzen der Spannmittel 23 auf die zweite Knochenschraube 2 wird das Verriegelungselement 25 bzgl. des Spannelements 24 quer ausgerichtet. Die Spannmittel 23 können dann in die U-förmige Ausnehmung 14 der zweiten Knochenschraube 2 eingefahren werden. Die Führungsrippen 27 und 28 des Spannelements 24 sind durch die Führungsflächen 20 und 21 der beiden Schenkel 17 und 18 geführt. Das Verriegelungselement 25 wird dann um die Achse der ersten Spannschraube 26 über die jeweilige Führungsrippe 18 verdreht, bis sich das Verriegelungselement 25 in der verriegelten Position befindet, wie dies aus Fig. 1 ersichtlich ist. Die erste Spannschraube 26 kann dann festgezogen werden und das zweite Verbindungselement 5 ist in der zweiten Knochenschraube 2 fixiert.

Wie aus Fig. 1 ersichtlich ist, bestehen die ersten Aufnahmemittel 7 der ersten Knochenschraube 6 jeweils aus zwei Schenkeln 45 und 46, welche eine U-Form bilden, in welche das erste Verbindungselement 8 einlegbar ist. Auf der Innenseite der Schenkel 45 und 46 ist ein Gewinde 47 angebracht. In dieses Gewinde 47 einschraubbar ist ebenfalls eine Spannschraube 48, mit welcher das erste Verbindungselement 8 in der ersten Knochenschraube 6 fixierbar ist. Selbstverständlich wäre es auch denkbar, die ersten Aufnahmemittel 7 der ersten Knochenschraube 6 in bekannter Weise anders auszugestalten, ebenfalls derart, dass das erste Verbindungselement 8 in den ersten Aufnahmemitteln 7 fixiert werden kann.

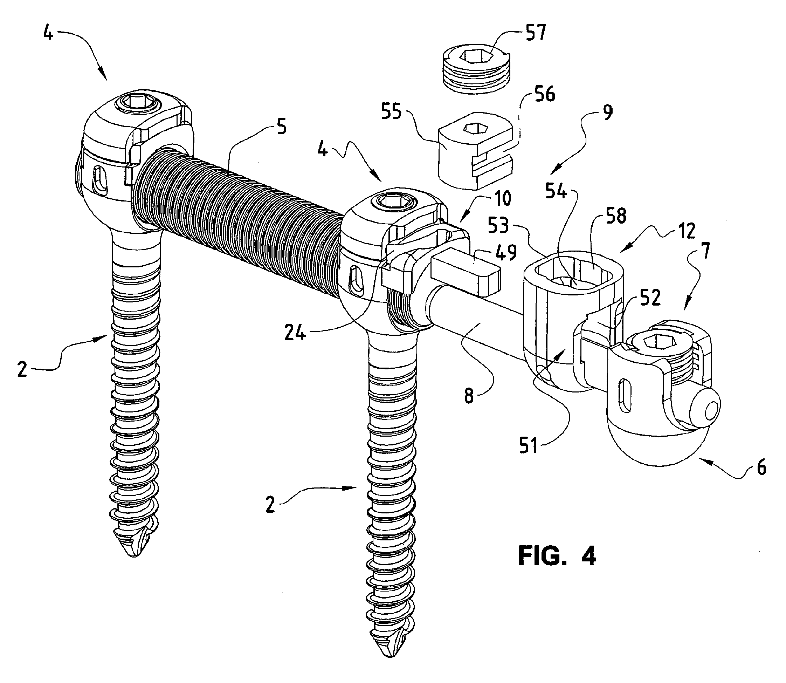

Aus den Fig. 4 bis 7 ist der Aufbau der in diesem ersten Ausführungsbeispiel eingesetzten Kupplungsmittel 9 dargestellt. Fig. 4 zeigt wiederum das Wirbelsäulenimplantat 1 gemäss Fig. 1 mit ersten Knochenschrauben 6 und ersten Aufnahmemitteln 7, in welchen das erste Verbindungselement 8 gehalten ist sowie zweiten Knochenschrauben 2 mit zweiten Aufnahmemitteln 4, in welchen das zweite Verbindungselement 5 gehalten ist.

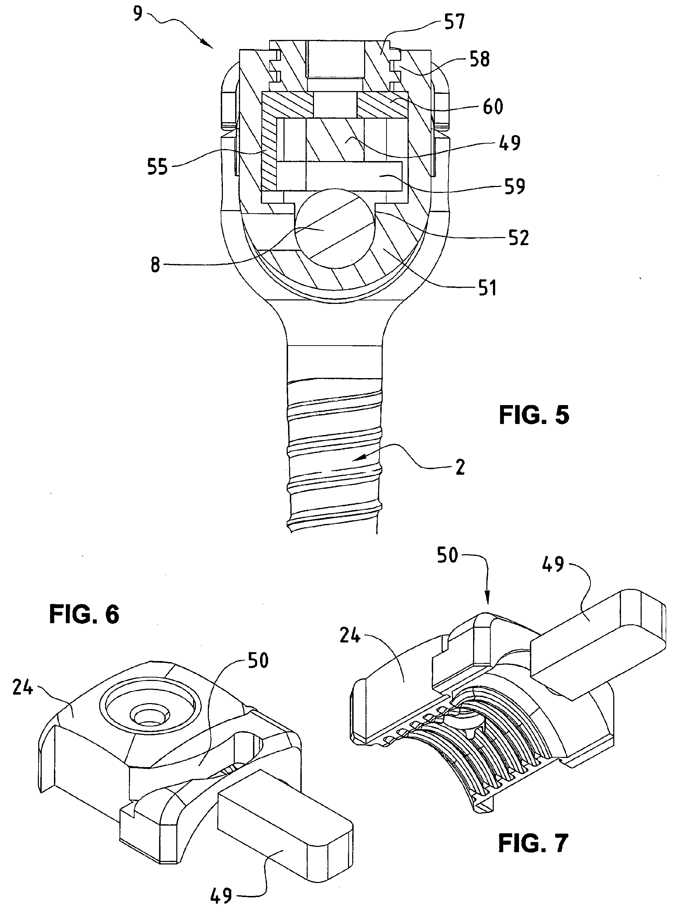

Der erste Bereich 10 der Kupplungsmittel 9 besteht aus einem Spannelement 24, wie es in den Fig. 2 und 3 dargestellt worden ist. Dieses Spannelement 24 ist Teil der Spannmittel 23, mit welchen eine zweite Knochenschraube 2 verschliessbar ist. Das Spannelement 24 ist hier mit einem nockenartigen Vorsprung 49 ausgestattet, der sich in Längsrichtung über den Endbereich 11 des zweiten Verbindungselements 5 hinaus erstreckt und parallel zum Endbereich 13 des ersten Verbindungselementes 8 verläuft. Dieses Spannelement 24 mit dem nockenartigen Vorsprung 49 ist im Detail aus den Fig. 6 und 7 ersichtlich. Um das Verriegelungselement 25, das in Fig. 2 dargestellt ist, bzgl. dieses Spannelements 24 verdrehbar zu machen, ist das Spannelement 24 im Bereich des nockenartigen Vorsprungs 49 mit einer schlitzförmigen Ausnehmung 50 versehen, die kreisbogenförmig verläuft.

Wie weiterhin aus Fig. 4 ersichtlich ist, ist der zweite Bereich 12 der Kupplungsmittel 9 ist als weiteres Spannmittel 51 ausgebildet, das eine U-förmige Aufnahme 52 für das erste Verbindungselement 8 aufweist. Die U-förmige Aufnahme 52 ist durch eine Abdeckung 53 verschlossen, welche mit einer Durchgangsöffnung 54 ausgestattet ist. Durch diese Durchgangsöffnung 54 ist ein Klemmelement 55 in das weitere Spannmittel 51 einsetzbar, das im weiteren Spannmittel 51 verdrehbar ist und mit einem Schlitz 56 ausgestattet ist. Das weitere Spannmittel 51 wird auf das erste Verbindungselement 8 aufgeschoben und das Klemmelement 55 wird durch die Durchgangsöffnung 54 in das weitere Spannmittel 51 eingesetzt und verdreht, so dass beim Verschieben dieses weiteren Spannmittels 51 mit eingesetztem Klemmelement 55 gegen den nockenartigen Vorsprung 49 des Spannelements 24 hin dieser nockenartige Vorsprung 49 in den Schlitz 56 einfährt. In das weitere Spannmittel 51 ist sodann eine zweite Spannschraube 57 einschraubbar, wozu das weitere Spannmittel 51 im Bereich der Durchgangsöffnung 54 mit einem entsprechenden Innengewinde 58 ausgestattet ist.

Aus Fig. 5 ist das weitere Spannmittel 51 im Zustand dargestellt, bei welchem das erste Verbindungsmittel 8 und der nockenartige Vorsprung 49 in diesem weiteren Spannmittel 51 fixiert sind. Hierbei ist ersichtlich, dass die untere Fläche des Klemmelements 55 auf das erste Verbindungselement 8 gepresst wird, welches sich am Grund der U-förmigen Aufnahme 52 abstützt. In den Schlitz 56 des Klemmelements 55 hinein ragt der nockenartige Vorsprung 49, der zwischen den beiden Schenkeln 59 und 60 des Klemmelementes 55, die sich elastisch gegeneinander bewegen können, klemmend gehalten ist. Über die zweite Spannschraube 57, die in das Innengewinde 58 des weiteren Spannmittels 51 eingeschraubt und festgezogen ist, werden die erforderlichen Klemmkräfte zum Halten des ersten Verbindungselements 8 und des nockenartigen Vorsprungs 49 im weiteren Spannmittel 51 aufgebracht.

Der grosse Vorteil dieses hier dargestellten Wirbelsäulenimplantats besteht darin, dass nach der Verwachsung der über das starre erste Verbindungselement 8 fixierten Wirbelkörper diese starre Fixierung vom durch das flexible zweite Verbindungselement 5 stabilisierenden Bereich für die Wirbelkörper vollständig entkoppelt werden kann. Dieser flexibel stabilisierte Bereich soll aber die stabilisierende Funktion weiterhin erfüllen. Zum Entkoppeln des starren Teils vom elastischen Teil des Wirbelsäulenimplantats kann die zweite Spannschraube 57 des weiteren Spannmittels 51 gelöst werden, das weitere Spannmittel 51 kann zusammen mit dem Klemmelement 55 entlang des ersten Verbindungselementes 8 von der zweiten Knochenschraube 2 weggeschoben werden, das Klemmelement 55 kann aus dem weiteren Spannmittel 51 herausgenommen werden. Danach können die Spannmittel 23 der den Endbereich 11 aufnehmenden zweiten Knochenschraube 2 gelöst werden, das Verriegelungselement 24 lässt sich dann aus dieser zweiten Knochenschraube 2 und axial dazu herausheben. Durch die formschlüssige Verbindung des zweiten Verbindungselements 5 mit der zweiten Knochenschraube 2 bleibt diese Verbindung stabil; auf die Knochenschraube 2 können dann ein Spannmittel 23 aufgesetzt werden, deren Spannelement 24 "normal" ohne nockenartigen Vorsprung 49 ausgebildet ist. Auf diese Weise kann somit der starre Bereich des Wirbelsäulenimplantats 1, nachdem dieser die Funktion erfüllt hat, vom elastischen Bereich vollständig entkoppelt werden, zwischen dem ersten Verbindungselement 8 und dem zweiten Verbindungselement 5 ergibt sich eine Lücke, sodass keine Kräfte mehr vom einen Verbindungselement auf das andere Verbindungselement übertragen werden können. Es ist auch denkbar, dass nach der Verwachsung der über das starre erste Verbindungselement 8 fixierten Wirbelkörper das starre erste Verbindungselement 8 und gegebenenfalls die ersten Knochenschrauben aus dem Körper des Patienten entfernt werden, was entsprechend über das Lösen der Kupplungsmittel 9 und ohne Beeinträchtigung des stabilisierenden, elastischen Bereichs des Wirbelsäulenimplantats erfolgen kann, der elastische Bereich des Wirbelsäulenimplantats bleibt unverändert im Körper.

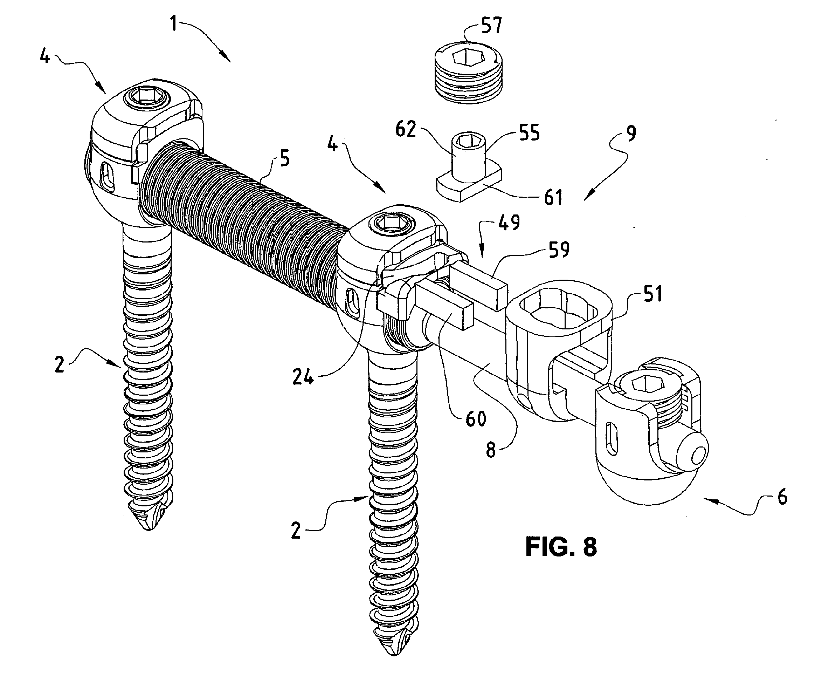

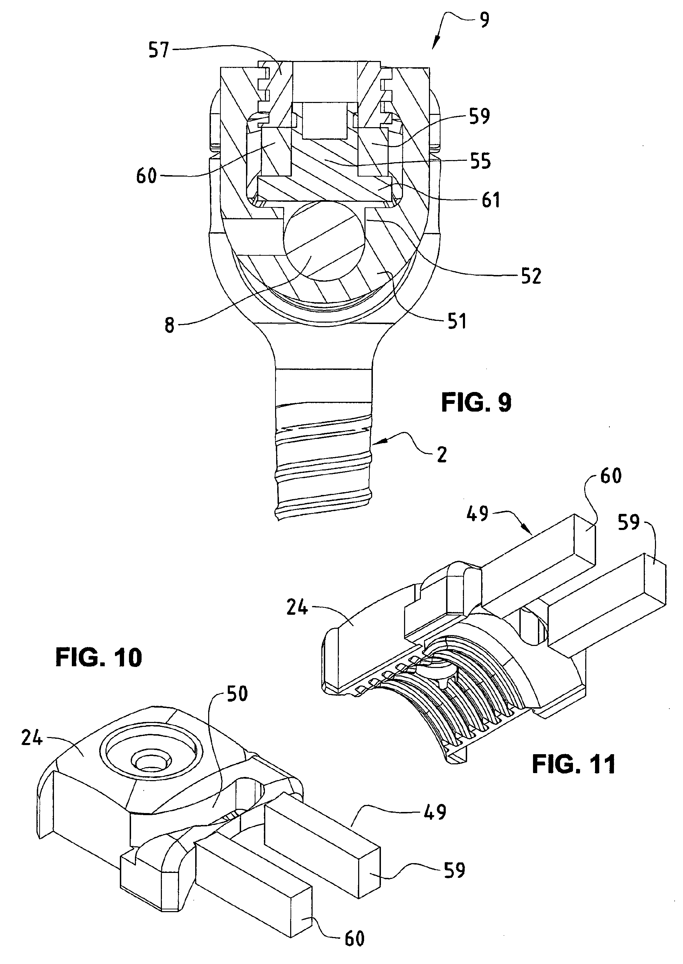

Die zweite Ausführungsform des erfindungsgemässen Wirbelsäulenimplantats 1, wie sie in den Fig. 8-11 dargestellt ist, unterscheidet vom vorgängig beschriebenen ersten Ausführungsbeispiel eines Wirbelsäulenimplantats lediglich dadurch, dass die Kupplungsmittel 9 anders ausgestaltet sind. Wie den Fig. 8, 10 und 11 entnehmbar ist, besteht der nockenartige Vorsprung 49, der am Spannelement 24 angebracht ist, aus zwei parallel verlaufenden Stegen 59 und 60. Das Klemmelement 55 setzt sich aus einer Grundplatte 61 und einem zylindrischen Teil 62 zusammen. Dieses Klemmelement 55 ist wiederum in das weitere Spannmittel 51 einsetzbar, die beiden Schenkel 59 und 60 gelangen wiederum in das weitere Spannmittel 51, mit der zweiten Spannschraube 57 kann das erste Verbindungselement 8 mit dem Spannelement 24 verbunden werden. Fig. 9 zeigt, dass im zusammengebauten Zustand das erste Verbindungselement 8 in der U-förmigen Aufnahme 52 eingelegt ist und sich darauf abstützt. Die Grundplatte 61 des Klemmelements 55 wird gegen das erste Verbindungselement 8 gepresst, indem die beiden Stege 59 und 60 des Klemmelements 55 über die zweite Spannschraube 57 gegen diese Grundplatte 61 gepresst werden. Dadurch wird auch hier eine optimale Fixierung des ersten Verbindungselements 8 mit dem Spannelement 24 erreicht. Das Entkoppeln des starren Bereichs des Wirbelsäulenimplantats vom elastischen Bereich des Wirbelsäulenimplantats ist auch hier, wie vorgängig beschrieben worden ist, in einfacher Weise möglich.

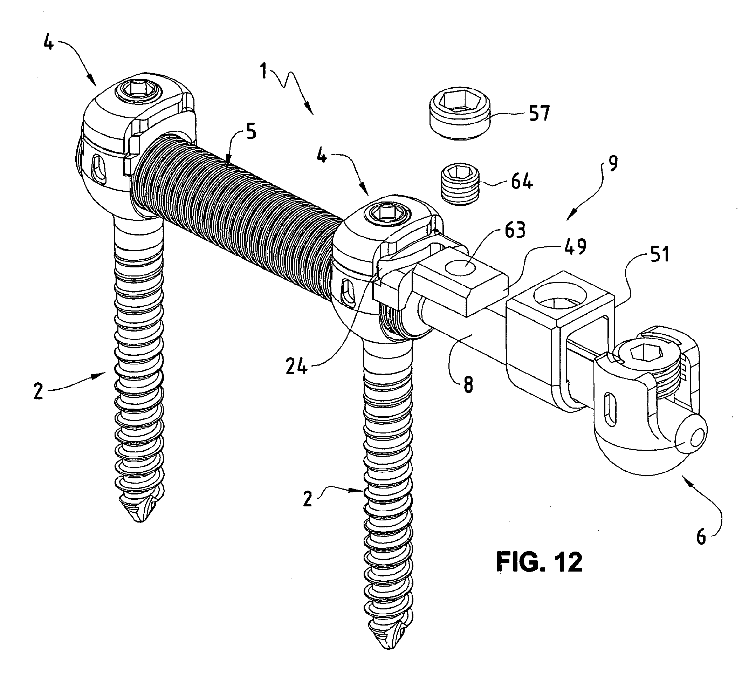

Das dritte Ausführungsbeispiel eines erfindungsgemässen Wirbelsäulenimplantats 1 ist in den Fig. 12-15 dargestellt. Dieses Ausführungsbeispiel unterscheidet sich wiederum durch ein anders ausgestaltetes Kupplungsmittel 9 gegenüber den beiden vorgängig beschriebenen Ausführungsbeispielen. Wie aus den Fig. 12, 14 und 15 ersichtlich ist, ist das Spannelement 24, das in die zweite Knochenschraube 2 einsetzbar ist, wiederum mit einem nockenartigen Vorsprung 49 versehen. Dieser ist gegenüber dem nockenartigen Vorsprung 49 gemäss dem ersten Ausführungsbeispiel breiter ausgestaltet. In diesem nockenartigen Vorsprung 49 ist eine Bohrung 63 angebracht, die mit einem Innengewinde versehen ist. In diese Gewindebohrung 63 ist eine weitere Spannschraube 64 einschraubbar.

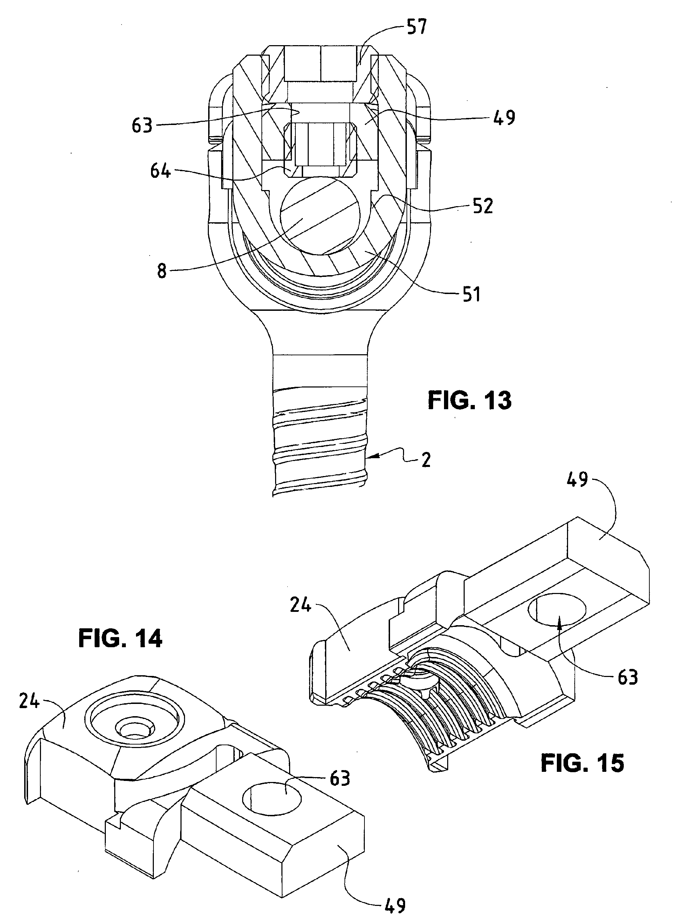

Im zusammengesetzten Zustand dieses Kupplungsmittel 9, wie dies aus Fig. 13 ersichtlich ist, befindet sich das erste Verbindungselement 8 in der U-förmigen Ausnahme 52 des weiteren Spannmittels 51. Der nockenartige Vorsprung 49 ragt in die U-förmige Aufnahme 52 des weiteren Spannmittels 51 hinein. Die in die Gewindebohrung 63 des nockenartigen Vorsprungs 49 eingeschraubte weitere Spannschraube drückt auf das erste Verbindungselement 8. Die zweite Spannschraube 57 drückt ihrerseits auf den nockenartigen Vorsprung 49. Dadurch ist das erste Verbindungselement 8 und der nockenartige Vorsprung 49 im weiteren Spannmittel 51 klemmend fixiert. Durch entsprechendes Einschrauben der weiteren Spannschraube 64 kann die Höhenlage des nockenartigen Vorsprungs 49 bzgl. des ersten Verbindungselements 8 eingestellt werden, wodurch in optimaler Weise Anpassungen der Position des ersten Verbindungselements 8 zu der benachbarten zweiten Knochenschraube 2 und demzufolge dem zweiten Verbindungselement 5 eingestellt werden kann.

Auch mit dieser Ausgestaltung des Kupplungsmittels 9 lässt sich der starre Bereich des Wirbelsäulenimplantats in einfachster Weise vom elastischen Stabilisierungsbereich des Wirbelsäulenimplantats, wie vorgängig beschrieben worden ist, entkoppeln, indem die zweite Spannschraube 57 und die weitere Spannschraube 64 herausgeschraubt werden.

Mit diesem erfindungsgemässen Wirbelsäulenimplantat wird, wie bereits erwähnt worden ist, der Vorteil erhalten, dass nach der Verknöcherung von Wirbelkörpern, die durch einen starren Bereich eines Wirbelsäulenimplantats erreicht wird, dieser starre Bereich in einfacher Weise vom elastischen Stabilisierungsbereich dieses Wirbelsäulenimplantats entkoppelt werden kann, dies würde gegebenenfalls auch eine einfache Herausnahme des starren Bereichs des Wirbelsäulenimplantats aus dem Körper ermöglichen, ohne dass der stabilisierende elastische Bereich des Wirbelsäulenimplantats beeinträchtigt wird.

## Patentansprüche

1. Wirbelsäulenimplantat zur Stabilisierung und Versteifung von Wirbelkörpern einer Wirbelsäule, umfassend erste Knochenschrauben (6), bestehend aus einem Einschraubteil (3), welcher in einen Wirbelkörper einschraubbar ist, und ersten Aufnahmemitteln (7), zweite Knochenschrauben (2), bestehend aus einem Einschraubteil (3), welcher in einen Wirbelkörper einschraubbar ist, und zweiten Aufnahmemitteln (4), erste Verbindungselemente (8), die starr sind und in die ersten Aufnahmemittel (7) der ersten Knochenschrauben (6) einsetzbar und darin befestigbar sind, zweite Verbindungselemente (5), die elastisch sind und in die zweiten Aufnahmemittel (4) der zweiten Knochenschrauben (2) einsetzbar und darin befestigbar sind, Kupplungsmittel (9), mit welchen jeweils ein erstes Verbindungselement (8) und ein zweites Verbindungselement (5) miteinander verbindbar sind, welche Kupplungsmittel (9) mit einem ersten Bereich (10) lösbar an einer zweiten Knochenschraube (2), in welcher ein Endbereich (11) eines zweiten Verbindungselementes (5) gehalten ist, befestigbar sind und mit einem dem ersten Bereich (10) gegenüberliegenden zweiten Bereich (12) mit einem Endbereich (13) eines ersten Verbindungselementes (8) verbindbar sind, und Spannmittel (23), wobei der zweite Bereich (12) des Kupplungsmittels (9) als weiteres Spannmittel (51) ausgebildet ist, in welchem ein Endbereich (13) eines ersten Verbindungselementes (8) befestigbar ist, **dadurch gekennzeichnet, dass** der erste Bereich (10) des Kupplungsmittels (9) mit einem Spannelement (24) versehen ist, welches in die zweiten Aufnahmemittel (4) geführt einsetzbar ist und über die Spannmittel (23), die auf die zweiten Aufnahmemittel (4) aufsetzbar sind, gegen das in die zweiten Aufnahmemittel (4) eingesetzte zweite Verbindungselement (5) spannbar ist.

2. Wirbelsäulenimplantat nach Anspruch 1, **dadurch gekennzeichnet, dass** die zweiten Aufnahmemittel (4) U-förmig ausgebildet sind und aus zwei Schenkeln (17, 18) bestehen, zwischen welchen die Auflagefläche (19) für das zweite Verbindungselement (8) angeordnet ist und auf welche zwei Schenkel (17, 18) die Spannmittel (23), bestehend aus einem mit einer ersten Spannschraube (26) versehenes Verriegelungselement (25), aufsetzbar und mit diesen verriegelbar ist.

3. Wirbelsäulenimplantat nach Anspruch 2, **dadurch gekennzeichnet, dass** das Spannelement (24) mit Führungsrippen (27, 28) ausgestattet ist, welche mit an den Schenkeln (17, 18) angebrachten Führungsflächen (20, 21) die Führungen bilden.

4. Wirbelsäulenimplantat nach einem der Ansprüche 1 bis 3, **dadurch gekennzeichnet, dass** das weitere Spannmittel (51) eine U-förmige Aufnahme (52) für das erste Verbindungselement (8) und eine der U-förmigen Aufnahme (52) gegenüberliegenden Gewindeteil (58) für die Aufnahme einer zweiten Spannschraube (57) aufweist.

5. Wirbelsäulenimplantat nach Anspruch 4, **dadurch gekennzeichnet, dass** zwischen die zweite Spannschraube (57) und das in das weitere Spannmittel (51) eingesetzten ersten Verbindungselement (8) ein Klemmelement (55) eingesetzt ist.

6. Wirbelsäulenimplantat nach einem der Ansprüche 1 bis 5, **dadurch gekennzeichnet, dass** das Spannelement (24) und das weitere Spannmittel (51), welche die Kupplungsmittel (9) bilden, lösbar miteinander verbunden sind.

7. Wirbelsäulenimplantat nach Anspruch 6, **dadurch gekennzeichnet, dass** das Spannelement (24) mindestens einen nockenartigen Vorsprung (49) aufweist, welcher in das weitere Spannmittel (51) hineinragt.

8. Wirbelsäulenimplantat nach Anspruch 7, **dadurch gekennzeichnet, dass** der nockenartige Vorsprung (49) mit einer Gewindebohrung (63) versehen ist, in welchen eine weitere Spannschraube (64) einschraubbar ist, welcher nockenartige Vorsprung (49) zwischen die zweite Spannschraube (57) und das erste Verbindungselement (8) des weiteren Spannmittels (51) hineinragt.

9. Wirbelsäulenimplantat nach einem der Ansprüche 1 bis 8, **dadurch gekennzeichnet, dass** die zweiten Verbindungselemente (5) eine Oberflächenstruktur aufweisen, die aus im Wesentlichen quer zur Längsachse verlaufenden Rippen (44) und Rillen (43) bestehen.

10. Wirbelsäulenimplantat nach Anspruch 9, **dadurch gekennzeichnet, dass** die zweiten Aufnahmemittel (4) und die dem zweiten Verbindungselement (5) zugewandte Fläche der Spannmittel (23) mit Rillen (42) und Rippen (41) versehen sind, die den Rippen (44) und Rillen (43) des zweiten Verbindungselementes (8) entsprechen.

## Claims

1. Vertebral column implant for stabilization and stiffening of vertebral bodies of a vertebral column, comprising:
first bone screws (6), consisting of a screw-in portion (3), which is screwable into a vertebral body, and first receiving means (7);
second bone screws (2), consisting of a screw-in portion (3), which is screwable into a vertebral body, and second receiving means (4);
first connecting elements (8), which are rigid and are insertable in the first receiving means (7) of the first bone screws (6) and are able to be fixed therein;
second connecting elements (5), which are elastic and are insertable in the second receiving means (4) of the second bone screws (2) and are able to be fixed therein;
coupling means (9), by means of which a first connecting element (8) and a second connecting element (5) are connectible to one another in each case,
which coupling means (9), with a first region (10), are able to be attached to a second bone screw (2) in a releasable way, in which second bone screw an end region (11) of a second connecting element (5) is held, and, with a second region (12) opposite the first region (10), are connectible to an end region (13) of a first connecting element (8), and tensioning means (23), whereby the second region (12) of the coupling means (9) is designed as further tensioning means (51), in which an end region (13) of a first connecting element (8) is able to be fixed, **characterised in that** the first region (10) of the coupling means (9) is provided with a tensioning element (24), which is insertable in a guided way into the second receiving means (4) and, via the tensioning means (23), which are able to be put on the second receiving means (4), is able to be tensioned against the second connecting element (5) inserted in the second receiving means (4).

2. Vertebral column implant according to claim 1, **characterised in that** the second receiving means (4) are designed U-shaped, and are composed of two arms (17, 18), between which the bearing surface (19) for the second connecting element (8) is disposed and on which two arms (17, 18) the tensioning means (23), consisting of a locking element (25) provided with a first tensioning screw (26), is able to be placed and locked therewith.

3. Vertebral column implant according to claim 2, **characterised in that** the tensioning element (24) is provided with guide ribs (27, 28), which, together with guide surfaces (20, 21) provided on the arms (17, 18), form the guides.

4. Vertebral column implant according to one of the claims 1 to 3, **characterised in that** the further tensioning means (51) has a U-shaped receiving part (52) for the first connecting element (8) and a threaded part (58), opposite the U-shaped receiving part (52), for receiving a second tensioning screw (57).

5. Vertebral column implant according to claim 4, **characterised in that** inserted between the second tensioning screw (57) and the first connecting element (8) inserted in the further tensioning means (51) is a clamping element (55).

6. Vertebral column implant according to one of the claims 1 to 5, **characterised in that** the tensioning element (24) and the further tensioning means (51), which form the coupling means (9), are connected to one another in a releasable way.

7. Vertebral column implant according to claim 6, **characterised in that** the tensioning element (24) has at least one nose-type projection (49), which projects into the further tensioning means (51).

8. Vertebral column implant according to claim 7, **characterised in that** the nose-type projection (49) is provided with a threaded bore (63), into which a further tensioning screw (64) is screwable, which nose-type projection (49) projects between the second tensioning screw (57) and the first connecting element (8) of the further tensioning means (51).

9. Vertebral column implant according to one of the claims 1 to 8, **characterised in that** the second connecting elements (5) have a surface structure consisting of ridges (44) and grooves (43) running substantially transversely to the longitudinal axis.

10. Vertebral column implant according to claim 9, **characterised in that** the second receiving means (4) and the surface of the tensioning means (23) turned toward the second connecting element (5) are provided with grooves (42) and ridges (41) that correspond to the ridges (44) and grooves (43) of the second connecting element (8).

## Revendications

1. Implant de colonne vertébrale pour la stabilisation et le raidissement des corps vertébraux d'une colonne vertébrale, comprenant des premières vis à os (6), consistant en une partie de vissage (3) qui est vissable dans un corps vertébral et des premiers moyens de réception (7), des deuxièmes vis à os (2), consistant en une partie de vissage (3) qui est vissable dans un corps vertébral et des seconds moyens de réception (4), des premiers éléments de jonction (8) qui sont rigides et insérables dans les premiers moyens de réception (7) des premières vis à os (6) et qui peuvent être fixés dans ceux-ci, des deuxièmes éléments de jonction (5) qui sont élastiques et insérables dans les deuxièmes moyens de réception (4) des deuxièmes vis à os (2) et qui peuvent être fixés dans ceux-ci, des moyens de couplage (9) avec lesquels un premier élément de connexion (8) et un second élément de connexion (5) respectivement peuvent être connectés l'un à l'autre, les moyens de couplage (9) pouvant être fixés de manière amovible par une première partie (10) à une seconde vis à os (2), dans laquelle est tenue une partie d'extrémité (11) d'un second élément de connexion (5), et pouvant être fixés par une seconde partie (12) opposée à la première partie (10) à une partie d'extrémité (13) d'un premier élément de connexion (8), et des moyens de tension (23), la seconde partie (12) du moyen de couplage (9) étant conçue comme un autre moyen de tension (51), dans lequel une partie d'extrémité (13) d'un premier élément de connexion (8) peut être fixé, **caractérisé en ce que** la première partie (10) du moyen de couplage (9) est munie d'un élément de tension (24), qui est insérable de manière guidée dans le second moyen de réception (4) et, par l'intermédiaire des moyens de tension (23) qui peuvent être placés sur le second moyen de réception (4) peut être tendu contre le second élément de connexion (5) inséré dans le second moyen de réception (4).

2. Implant de colonne vertébrale selon la revendication 1, **caractérisé en ce que** les seconds moyens de réception (4) sont conçus en forme de U et sont composés de deux bras (17, 18), entre lesquels la surface d'appui (19) pour le second élément de connexion (8) est disposée et sur lesquels deux bras (17, 18) les moyens de tension (23), consistant en un élément de verrouillage (25) muni d'une première vis de tension (26), peut être placé et verrouillé par ceux-ci.

3. Implant de colonne vertébrale selon la revendication 2, **caractérisé en ce que** l'élément de tension (24) est prévu avec des nervures de guidage (27, 28), qui forment les guides avec les surfaces de guidage (20, 21) prévues sur les bras (17, 18).

4. Implant de colonne vertébrale selon l'une quelconque des revendications 1 à 3, **caractérisé en ce que** l'autre moyen de tension (51) a une partie de réception (52) en forme de U pour le premier élément de connexion (8) et une partie filetée (58), opposée à la partie de réception en forme de U (52), pour la réception d'une seconde vis de tension (57).

5. Implant de colonne vertébrale selon la revendication 4, **caractérisé en ce qu'**un élément de serrage (55) est inséré entre la seconde vis de tension (57) et le premier élément de connexion (8) inséré dans l'autre moyen de tension (51).

6. Implant de colonne vertébrale selon l'une quelconque des revendications 1 à 5, **caractérisé en ce que** l'élément de tension (24) et l'autre moyen de tension (51) qui forment les moyens de couplage (9), sont connectés l'un à l'autre de manière amovible.

7. Implant de colonne vertébrale selon la revendication 6, **caractérisé en ce que** l'élément de tension (24) a au moins une saillie en forme de came (49), qui se projette dans l'autre moyen de tension (51).

8. Implant de colonne vertébrale selon la revendication 7, **caractérisé en ce que** la saillie en forme de came (49) est munie d'un alésage fileté (63), dans lequel une autre vis de tension (64) est vissable, laquelle saillie en forme de came (49) se projette entre la seconde vis de tension (57) et le premier élément de connexion (8) de l'autre moyen de tension (51).

9. Implant de colonne vertébrale selon l'une quelconque des revendications 1 à 8, **caractérisé en ce que** les seconds éléments de connexion (5) ont une structure de surface consistant en des nervures (44) et des rainures (43) s'étendant substantiellement transversalement à l'axe longitudinal.

10. Implant de colonne vertébrale selon la revendication 9, **caractérisé en ce que** les seconds moyens de réception (4) et la surface du moyen de tension (23) tournée en direction du second élément de connexion (5) sont munis de rainures (42) et de nervures (41) qui correspondent aux nervures (44) et rainures (43) du second élément de connexion (8).
